# EUROPÄISCHE PATENTSCHRIFT

(11) **EP 1 002 496 B1**
(45) Veröffentlichungstag und Bekanntmachung des Hinweises auf die Patenterteilung: **09.04.2003**
(21) Anmeldenummer: 98811141.5
(22) Anmeldetag: 17.11.1998
(51) Int. Cl.: A61B 5/00

(54) **Verfahren und Vorrichtung zur Fernüberwachung von Körperfunktionen**
Method and device for remote monitoring of body functions
Procédé et dispositif de surveillance à distance de fonctions corporelles

(43) Veröffentlichungstag der Anmeldung: 24.05.2000
(73) Patentinhaber: Schiller AG, 6340 Baar (CH)
(72) Erfinder: Schiller, Alfred E., 8914 Aeugst a. A, (CH); Schmid, Johann-Jakob, 8911 Rifferswil (CH)
(74) Vertreter: Hepp, Dieter

(56) Entgegenhaltungen:
- EP-A- 0 790 034
- EP-A- 0 846 440
- WO-A-97/28736
- WO-A-97/48244
- WO-A-97/49077
- DE-C- 19 707 681
- FR-A- 2 727 850
- US-A- 5 544 661

## Beschreibung

Die Erfindung betrifft ein Verfahren und eine Vorrichtung zur Fernüberwachung von Körperfunktionen mit den Merkmalen des Oberbegriffs der unabhängigen Patentansprüche.

Es ist bekannt, Körperfunktionen, insbesondere Herz- und Kreislauffunktionen von Risikopatienten kontinuierlich zu überwachen. Insbesondere ist es bekannt, am Körper Messparameter wie EKG-Werte, Blutdruck oder ähnliches zu erfassen und auszuwerten. Zur Untersuchung von Patienten in Krankenhäusern ist es bekannt, am Körper befestigte Messanordnungen zu verwenden, welche die gemessenen Parameter drahtlos zu einer Erfassungsstelle im Krankenhaus übermitteln.

Ein Nachteil bei diesem Verfahren besteht darin, dass der Patient sich immer in der Nähe des Krankenhauses aufhalten muss und dass die Überwachung nicht in seiner gewohnten Umgebung durchgeführt werden kann. Die mit einer solchen Überwachung gewonnenen Werte entsprechen also nicht unbedingt den im täglichen Leben auftretenden Werten. Vor allem für die Bestimmung von Risikopatienten ist also dieses bekannte Messverfahren zu wenig aussagekräftig.

Aus WO-A-97/49077 ist ein Verfahren bzw. eine Vorrichtung zur Fernüberwachung von Körperfunktionen bekannt. Dazu werden Messanordnungen zum Erfassen von medizinisch relevanten Parametern verwendet und ist eine Sende- und Empfangsstation zur Übertragung der Messparameter an eine Erfassungsstelle vorgesehen.

Es ist deshalb eine Aufgabe der Erfindung, die Nachteile des Bekannten zu vermeiden, insbesondere also ein Verfahren und eine Vorrichtung zur Fernüberwachung von Körperfunktionen zu schaffen, welches dem Patienten erlaubt, sich in seiner normalen Umgebung aufzuhalten und ein normales Leben zu führen. Das Verfahren soll eine zuverlässige Übermittlung der erfassten Messparameter gewährleisten, den Patienten in seiner Bewegungsfreiheit so wenig als möglich einschränken und auf einfache Weise betreibbar sein.

Erfindungsgemäss werden diese Aufgaben mit einem Verfahren und einer Vorrichtung mit den Merkmalen des kennzeichnenden Teils der unabhängigen Patentansprüche gelöst.

In einem Verfahren zur Fernüberwachung von Körperfunktionen werden medizinisch relevante Messparameter am Körper mittels Sensoren einer Messanordnung erfasst. Bei den überwachten Körperfunktionen handelt es sich insbesondere um Herz- und Kreislauffunktionen. Die gemessenen Werte sind typischerweise ein EKG, EEG, der Blutdruck oder der Puls. Gleichzeitig können weitere Werte wie Stoffkonzentrationswerte in Körperflüssigkeiten, Temperaturen, Atmungswerte oder der Hautwiderstand gemessen werden. Ausserdem kann auch die Bewegung des Patienten erfasst werden.

Die ermittelten Messparameter werden durch eine mit der Messanordnung verbundene oder verbindbare Sende- und Empfangsanordnung direkt oder indirekt zu einer Erfassungsstelle übertragen. Die Erfassungsstelle speichert die übertragenen Messparameter und wertet diese aus.

Gemäss den Merkmalen der Erfindung werden die Messparameter drahtlos zu einer Empfangs- und Sendestation übermittelt. Die Empfangs- und Sendestation ist im allgemeinen nicht identisch mit der Erfassungsstelle. Die Messparameter werden von der Empfangs- und Sendestation beispielsweise über ein normales Telefonnetz zu der Erfassungsstelle übermittelt.

Aus einer Mehrzahl von zur Verfügung stehenden Empfangs- und Sendestationen wird erfindungsgemäss eine für die Übertragung geeignete Empfangs- und Sendestation ausgewählt. Insbesondere wird diejenige Empfangs- und Sendestation ausgewählt, die in der Sende- und Empfangsanordnung der Messanordnung das stärkste Signal erzeugt.

In einer bevorzugten Ausführungsform der Erfindung wird jeweils automatisch diejenige Empfangs- und Sendestation ausgewählt, die in der Sende- und Empfangsanordnung das stärkste Signal erzeugt

Die Empfangs- und Sendestationen sind vorzugsweise bestehende Stationen eines Telekommunikationsnetzwerkes, beispielsweise Stationen eines GSM, eines UMTS-Netzwerkes oder Telekommunikationssatelliten. Es ist aber auch denkbar, dass die Übertragung zeitweise über lokale Funknetzwerke erfolgt, beispielsweise wenn sich der Patient im Krankenhaus, bei sich zu Hause oder am Arbeitsplatz befindet.

Die Sende- und Empfangsanordnung, die mit der Messanordnung verbunden ist, kann beispielsweise aus einem handelsüblichen GSM-Mobiltelefon bestehen. Das Mobiltelefon sucht sich jeweils die am nächsten liegende Empfangs- und Sendestation aus. Gleichzeitig ist es denkbar, dass die Sende- und Empfangsanordnung bei fehlendem Empfang in einem GSM-Netz die ermittelten Messparameter über eine Satellitenverbindung übermittelt. Die Sende- und Empfangsanordnung wählt also erfindungsgemäß aus einer Mehrzahl von verschiedenen Übertragungsmedien, insbesondere GSM-Netzen, Satellitenverbindungen und lokalen Funknetzen aus.

Es hat sich gezeigt, dass vor allem bei der Übertragung in GSM-Netzen mit handelsüblichen Mobiltelefonen Probleme bei der Zuverlässigkeit der Übertragung auftreten. Die Verbindung wird bei solchen herkömmlichen Mobiltelefonen nach einer bestimmten Datenmenge unterbrochen. Diese Datenmenge ist für die Übertragung der gemessenen Parameter häufig nicht ausreichend. In einem bevorzugten Ausführungsbeispiel der Erfindung erfolgt die Übertragung der Messparameter mit dem Radio-Link-Protokoll (RLP).

Die Messparameter können ausserdem vor der Übertragung durch Codierung, Kompression und/oder Interleaving vorbereitet werden. Codierung erfolgt aus Sicherheitsgründen, Kompression zur Erhöhung der Übertragungsgeschwindigkeit. Beim Interleaving werden die Daten in nicht chronologischer Reihenfolge übermittelt. Eine Störung der Verbindung führt also nicht zum Verlust von ganzen Datenblöcken sondern allenfalls zu einer Reduktion der Übertragungsqualität.

Die Messparameter können von der Messanordnung erfasst, in dieser zwischengespeichert und von der Sende- und Empfangsanordnung übertragen werden. Insbesondere werden die Messparameter periodisch, beispielsweise alle 30 Minuten übertragen.

Es ist aber auch denkbar, die Messparameter bei Bedarf zu übertragen, beispielsweise wenn der Benutzer des Verfahrens es wünscht.

In einem anderen Ausführungsbeispiel werden die Messparameter von der Messanordnung erfasst, in dieser zwischengespeichert und von der Erfassungsstelle abgefragt. Die Abfragung kann periodisch aber auch im On-line-Betrieb erfolgen.

Bei der Erfassungsstelle handelt es sich typischerweise um einen zentralen Rechner, der gleichzeitig eine Mehrzahl von verschiedenen Messanordnungen kontrolliert. Es ist auch denkbar, dass verschiedene Erfassungsstellen eingerichtet sind, wobei bei Überlastung einer Erfassungsstelle die erfassten Parameter an eine nächste Erfassungsstelle weiterleitet.

Es ist denkbar in der Erfassungsstelle und/oder in der Messanordnung einen Alarm auszulösen, sobald wenigstens einer der erfassten Messparameter einen vorbestimmbaren Grenzwert übersteigt.

Die übermittelten Messparameter können in der Erfassungsstelle in zentralen Speichermitteln gespeichert werden.

Vorteilhaft kann bei der Verringerung der Stärke des Signals einer ersten Empfangs- und Sendestation und bei gleichzeitiger Zunahme des Signals einer zweiten Empfangs- und Sendestation während der Übertragung der Messparameter die Verbindung von der ersten Empfangs- und Sendestation zu der zweiten Empfangs- und Sendestation übergeben werden. Dabei kann es sich um verschiedene Empfangs- und Sendestationen eines GSM-Netzwerk, aber auch um Empfangs- und Sendestationen von verschiedenen Netzwerken, beispielsweise einem GSM-Netzwerk und einem Satellitennetzwerk handeln.

Die Entscheidung, welche Empfangs- und Sendestation für die Übertragung verwendet werden soll, muss nicht alleine von der Stärke des empfangenen Signals abhängen. Es ist auch denkbar, weitere Kriterien wie Übertragungskosten, Übertragungsgeschwindigkeit oder Zuverlässigkeit zu berücksichtigen.

Die erfindungsgemässe Vorrichtung zur Fernüberwachung von Körperfunktionen wie beispielsweise Herz- und Kreislauffunktionen besteht im wesentlichen aus einer Messanordnung mit wenigstens einem Sensor zum Erfassen eines medizinisch relevanten Parameters insbesondere EKG, EEG, Blutdruck oder ähnliches und aus einer mit der Messanordnung verbundenen oder verbindbaren Sende- und Empfangsanordnung. Die Sendeund Empfangsanordnung ist zum Übermitteln der erfassten Messparameter an eine aus einer Mehrzahl von Empfangs- und Sendestationen auswählbaren Empfangs- und Sendestation ausgebildet. Die Sende- und Empfangsanordnung ist beispielsweise zum Übermitteln der Messparameter via GSM, UMTS oder Satellitennetzwerk und vorzugsweise auch über ein lokales Funknetzwerk ausgebildet.

Die Sende- und Empfangsanordnung kann derart ausgebildet sein, dass sie das Signal via Radio-Link-Protocol (RLP) übertragen kann.

Die Vorrichtung kann ausserdem mit Rechenmitteln zum Bearbeiten der Messparameter, insbesondere zum Codieren, Komprimieren oder zum Erzeugen eines Interleavings, ausgerüstet sein.

Die Vorrichtung kann ausserdem Speichermittel zum zeitweisen Speichern der ermittelten Messparameter aufweisen.

Das erfindungsgemässe Verfahren bzw. die Vorrichtung können zu verschiedenen Zwecken eingesetzt werden. Beispielsweise ist die dauernde Überwachung von Risikopatienten möglich. Weiter ist es denkbar, die Vorrichtung zum Überwachen von Patienten nach einem operativen Eingriff einzusetzen. Schliesslich kann die Vorrichtung auch dazu verwendet werden, einen Patienten im täglichen Leben zu überwachen und zu bestimmen, ob Risikofaktoren vorhanden sind.

Die Erfindung wird im Folgenden in einem Ausführungsbeispiel und anhand der Zeichnungen näher erläutert: Es zeigen
- Figur 1: eine schematische Darstellung der Übertragung der Messparameter und
- Figur 2: eine schematische Darstellung der erfindungsgemässen Messanordnung.

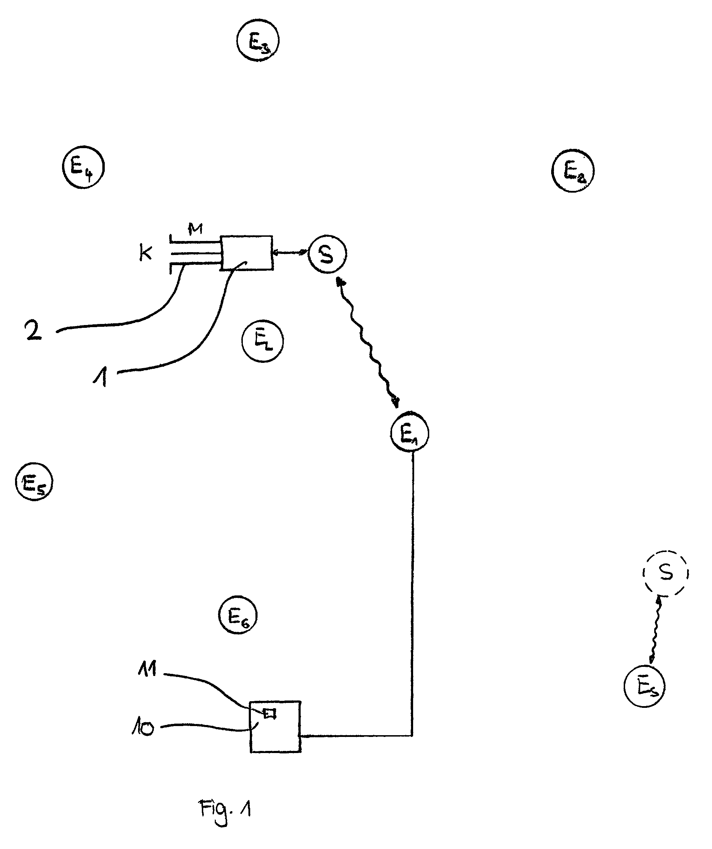

In Figur 1 ist schematisch eine Messanordnung 1 zum Erfassen von medizinisch relevanten Messparametern M an einem Körper K gezeigt. Die Messanordnung 1 ist mit einer Vielzahl von Messsensoren 2 versehen, die zum Messen von verschiedenen Daten dienen. Die Messanordnung 1 ist ausserdem mit einer Sende- und Empfangsanordnung S versehen. Die Sende- und Empfangsanordnung S dient zum drahtlosen Übermitteln der ermittelten Messparameter M an eine aus einer Vielzahl von bestehenden Empfangs- und Sendestationen ausgewählten Empfangs- und Sendestation E₁. Zur Übermittlung der Messparamter M werden Empfangs- und Sendestationen E₁ bis E₆, E₁ und Eₛ von bestehenden Funknetzwerken verwendet. In Figur 1 stellen E₁ bis E₆ verschiedene Empfangs- und Sendestationen eines GSM-Netzes dar. Die Sende- und Empfangsanordnung S stellt fest, zu welcher der Empfangs- und Sendestationen E₁ bis E₆ der beste Empfang besteht. Die Übertragung der Messparameter M erfolgt drahtlos zu dieser Empfangs- und Sendestation E₁. Wenn sich die Messanordnung 1 bewegt, können sich die Empfangsverhältnisse ändern. Sobald die Verbindung mit einer anderen Empfangs- und Sendestation besser wird, kann die Verbindung zwischen der Sende- und Empfangsanordnung in der Messanordnung 1 an eine andere Empfangs- und Sendestation übergeben werden.

Das drahtlos an die Empfangs- und Sendestation E₁ übermittelte Signal wird von da zu einer zentralen Erfassungsstelle 10 übermittelt. Üblicherweise erfolgt diese Übermittlung über ein normales, drahtgebundenes Telekommunikationsnetz.

Wenn sich die Messanordnung 1 in der Nähe eines lokalen Funknetzwerkes (beispielsweise betriebsinterne Funktelefone) befindet, kann die Sende- und Empfangsanordnung S der Messanordnung 1 auch Verbindung zu einer Empfangs- und Sendestation E₁ aufnehmen.

Wenn kein Empfang zu den Empfang- und Sendestation E₁ bis E₆ eines GSM-Netzwerkes möglich ist, erfolgt die Datenübertragung über eine Empfangs- und Sendestation Eₛ eines Telekommunikationssatelliten.

Lokale Funknetzwerke sind in einem Bereich zwischen 200 m und max. etwa 10 km einsetzbar. GSM-Netzwerke sind bei entsprechender Abdeckung über 10 bis 5'000 km einsetzbar. Satellitenunterstützte Datenübertragung ist weltweit uneingeschränkt möglich, sowohl am Boden als auch in der Luft.

Die Messanordnung 1 entscheidet jeweils automatisch, welche der zur Verfügung stehenden Empfangs- und Sendestationen für die Übermittlung verwendet werden soll.

Die zentrale Erfassungsstelle 10 dient zum Steuern der Messanordnung 1 und zum Überwachen und gegebenenfalls Speichern der erfassten Messparameter M. Die Erfassungsstelle 10 kann ein Speichermedium 11 aufweisen, in welchem die erfassten Messparameter zentral gespeichert werden.

Bei Inbetriebnahme der Messanordnung 1 nimmt der Benutzer der Messanordnung 1 mit der zentralen Erfassungsstelle 10 Kontakt auf. Dies kann via Sprechverbindung oder auch via Datenverbindung erfolgen. Die Erfassungsstelle 10 kann die Messanordnung 1 via Datenübertragung für den Betrieb vorbereiten. Insbesondere kann beispielsweise die Position der Messsensoren 2 überprüfen (z.B. Elektrodenposition zum Bestimmen eines EKG) oder Grenzwerte einstellen. Je nach gemessenem Messparameter und auch in Abhängigkeit des Benutzers können verschiedene Bereiche für die einzelnen Messparameter festgelegt werden. Falls Messparameter ausserhalb dieser Bereiche liegen, wird in der Messanordnung 1 und/oder in der zentralen Erfassungsstelle 10 ein Alarm ausgelöst.

Die zentrale Erfassungsstelle 10 kann eine grössere Anzahl von Messanordnungen 1 bedienen.

Alle sich in Betrieb befindlichen Messanordnungen werden der Reihe nach in vorbestimmbaren Intervallen abgefragt. Die mit den Messanordnung 1 erfassten Messparameter werden im zentralen Speichermedium 11 abgelegt. Die verarbeiteten Daten können von einem Arzt ausgewertet werden.

Es ist zusätzlich möglich, von der Messanordnung 1 zu jedem beliebigen Zeitpunkt eine Verbindung mit der Erfassungsstelle 10 aufzubauen. Dies kann insbesondere bei einem Unwohlsein des Benutzers der Messanordnung 1 vorteilhaft sein. Ausserdem ist es möglich, die mit der Messanordnung 1 erfassten Messparameter M im On-line-Betrieb zu beobachten.

Das zentrale Speichermedium 11 hat den Vorteil, dass die Daten auch bei einem Stromunterbruch in der Messanordnung 1 nicht verloren gehen.

Bei fehlerhafter Verbindung zwischen der Messanordnung 1 und der Erfassungsstelle 10 (vor allem bei schlechtem oder fehlendem Empfang bei der drahtlosen Übertragung) kann ausserdem eine Warnung in der Messanordnung 1 und/oder der Erfassungsstelle 10 ausgegeben werden.

Wenn die Erfassungsstelle 10 überlastet ist kann die Verbindung automatisch einer alternativen Empfangsstelle 10' (in Figur 1 nicht gezeigt) übergeben werden.

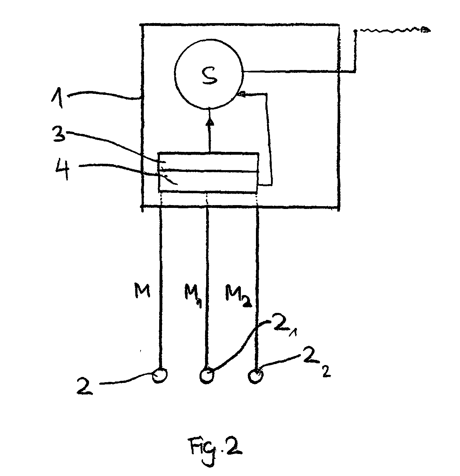

In Figur 2 ist schematisch eine Messanordnung 1 etwas detaillierter dargestellt. Die Messanordnung 1 weist eine Mehrzahl von Sensoren 2, 2₁, 2₂ auf, welche zum Erfassen von Messparametern M, M₁ und M₂ dienen. Typischerweise werden die folgenden Messparameter ermittelt:
- Elektrische Potentiale, insbesondere EKG, EEG, EMG
- Stoffkonzentrationswerte, beispielsweise ETO₂, Partialdruck CO₂, pH-Wert, Glucose-Konzentration
- Drücke im Kreislaufsystem, insbesondere systolischer, diastolischer und mittlerer Blutdruck sowie Atmungsdrükke
- Temperaturen
- Atmungswerte, insbesondere Atmungsfrequenz, Fluss, Volumen, Impedanz
- Hautwiderstand
- weitere Messparameter, beispielsweise Bewegung des Benutzers der Messanordnung 1

Die Parameter werden durch an sich dem Fachmann bekannte Messsensoren gemessen. Die Messung der Messparameter M, M₁, M₂ erfolgt über eine Rechneranordnung 4, die in der Messanordnung 1 integriert ist. Die Rechneranordnung 4 ermöglicht gleichzeitig auch eine Vermessung und Interpretation der erfassten Messparameter und gegebenenfalls eine Alarmierung. Die erfassten Messparameter können in einem Speichermedium 3 zwischengespeichert werden.

Die Messanordnung 1 ist ausserdem mit einer Sende- und Empfangsanordnung S versehen. Die Sende- und Empfangsanordnung S ist gleichzeitig zur Übertragung von Daten via lokales Funknetzwerk, via GSM (oder auch UMTS) und via Satellitenfunk ausgebildet. Die Rechneranordnung 4 kontrolliert auch den Betrieb der Sende- und Empfangsanordnung S. Insbesondere wird automatisch der jeweils günstigste Übertragungsmodus ermittelt. Kriterien sind:
- Ermittelte Feldstärke
- Kosten
- Geschwindigkeit

Die Rechneranordnung 4 dient ausserdem zum Vorbereiten der die Werte der Messparameter M, M₁, M₂ representierenden Daten für die Übermittlung.

Die ermittelten Daten werden insbesondere komprimiert und codiert. Gegebenenfalls ist zur Verbesserung der Übertragungsqualität auch Interleaving denkbar.

Die in Figur 1 gezeigte Erfassungsstelle 10 ist mit ähnlichen Rechnermitteln zum Decodieren, Dekomprimieren und Zusammensetzen der übermittelten Signale ausgebildet.

Die die Werte der Messparameter enthaltenden Daten werden mit dem Radio-Link-Protocol (RLP) übertragen. Damit wird eine zuverlässige Übertragung auch bei grösseren Datenmengen gewährleistet.

Eine Verbindung zwischen der zentralen Erfassungsstelle 10 und der Messanordnung 1 ist in beide Richtungen möglich. Damit ist sowohl eine Betreuung und Überwachung des Benutzers der Messanordnung 1 von der Erfassungsstelle 10 aus als auch Hilferuf des Benutzers an die Erfassungsstelle 10 möglich.

Die Erfassungsstelle 10 kann in einen Verbund von verschiedenen Stellen eingebunden sein und von medizinischen Fachkräften betreut sein. Bei Überlastung der Station wird die Kontrolle von einer weiteren, noch Kapazität aufweisenden Erfassungsstelle betreut.

Zum Ermöglichen einer Notruffunktion sind in der Messanordnung 1 die Telefonnummern der zentralen Erfassungsstelle 10 sowie evtl. der Ausweichzentralen erfasst. Beim Auslösen eines Notrufs wird der Notruf automatisch durch die Zentrale entgegengenommen oder an eine Ausweichzentrale weitergeleitet. Gleichzeitig wird ein Arzt durch die Zentrale alarmiert. Sofern nicht innerhalb einer bestimmten Zeit eine Quittierung durch den Arzt erfolgt, wird der Notruf an die Ausweichzentrale weitergeleitet.

Wenn keine Quittierung erfolgt, kann gleichzeitig ein Alarmsignal in der Messanordnung 1 ausgelöst werden.

Die Sende- und Empfangsanordnung S besteht überlicherweise aus einem handelsüblichen Mobiltelefon, welches GSM-Netze und Satelliten-Netze und vorzugsweise auch lokale Funknetzwerke unterstützt. Allenfalls nötige Änderungen im Übertragungsprotokoll können softwareseitig durchgeführt werden.

## Patentansprüche

1. Verfahren zur Fernüberwachung von Körperfunktionen, insbesondere Herz- und Kreislauffunktionen, enthaltend die Schritte
Erfassen von medizinisch relevanten Parametern (M, M₁, M₂), mittels Sensoren einer Messanordnung (1) an einem Körper (K)
und Übertragen der ermittelten Messparameter (M) durch eine mit der Messanordnung (1) verbundenen oder verbindbaren Sende- und Empfangsanordnung (S) an eine Erfassungsstelle (10),
wobei die Messparameter (M, M₁, M₂) drahtlos zu einer Empfangs- und Sendestation (E₁ bis E₆, E₁, Eₛ) und von dieser zu der Erfassungsstelle (10) übermittelt werden, und
wobei die Empfangs- und Sendestation (E₁) aus einer Mehrzahl von zur Verfügung stehenden Empfangs- und Sendestationen (E₁ bis E₆, E₁, Eₛ) ausgewählt wird, **dadurch gekennzeichnet, dass** die drahtlose Übertragung wahlweise mittels einem aus einer Mehrzahl von Übertragungsmedien ausgewählten Medium, insbesondere mittels GSM- oder UMTS-Netzwerk oder mittels einer Satellitenverbindung oder eines lokalen Funknetzwerks erfolgt.

2. Verfahren nach Anspruch 1, **dadurch gekennzeichnet, dass** zum Übertragen der Messparameter (M, M₁, M₂) automatisch die in der Sende- und Empfangsanordnung (S) das stärkste Signal erzeugende Empfangs- und Sendestation (E) ausgewählt wird.

3. Verfahren nach Anspruch 1, **dadurch gekennzeichnet, dass** die Übertragung über RLP erfolgt.

4. Verfahren nach einem der Ansprüche 1 bis 3, **dadurch gekennzeichnet, dass** vor der Übertragung die zu übertragenden Daten durch Codierung, Kompression und/oder Interleaving vorbereitet werden.

5. Verfahren nach einem der Ansprüche 1 bis 4, **dadurch gekennzeichnet, dass** die Messparameter von einer auf dem Körper (K) getragenen Messanordnung (1) erfasst, in der Messanordnung (1) in Speichermitteln (3) zwischengespeichert und zeitweise übermittelt werden.

6. Verfahren nach einem der Ansprüche 1 bis 4, **dadurch gekennzeichnet, dass** die Messparameter von einer auf dem Körper (K) getragenen Messanordnung (1) erfasst und in der Messanordnung (1) in Speichermitteln (3) zwischengespeichert werden und dass die im Zwischenspeicher (3) gespeicherten Daten von der Erfassungsstelle (10) abgefragt werden.

7. Verfahren nach einem der Ansprüche 1 bis 6, **dadurch gekennzeichnet, dass** wenigstens einer der Messparameter (M, M₁, M₂) aus der Gruppe elektrische Potentiale im Körper, Stoffkonzentrationen in Körperflüssigkeiten, Drücke im Kreislaufsystem, Körpertemperatur, Atmungsparameter, Hautwiderstand oder Körperbewegung erfasst und übermittelt wird.

8. Verfahren nach einem der Ansprüche 1 bis 7, **dadurch gekennzeichnet, dass** in der Erfassungsstelle (10) und/oder der Messanordnung (1) ein Alarm ausgelöst wird, sobald wenigstens einer der erfassten Parameter (M, M₁, M₂) einen vorbestimmbaren Grenzwert übersteigt.

9. Verfahren nach einem der Ansprüche 1 bis 8, **dadurch gekennzeichnet, dass** die übermittelten Messparameter (M, M₁, M₂) in der Erfassungsstelle (10) in einem zentralen Speichermittel (11) gespeichert werden.

10. Verfahren nach einem der Ansprüche 1 bis 9, **dadurch gekennzeichnet, dass** bei Verringerung der Stärke des Signals einer ersten Empfangs- und Sendestation (E₁) und bei Zunahme des Signals einer zweiten Empfangs- und Sendestation (E₂) während der Datenübertragung die Verbindung von der ersten Empfangs- und Sendestation (E₁) auf die zweite Empfangs- und Sendestation (E₂) übergeben wird.

11. Vorrichtung zur Fernüberwachung von Körperfunktionen, insbesondere Herz- und Kreislauffunktionen,
mit einer Messanordnung (1) mit wenigstens einem Sensor (2, 2₁, 2₂) zum Erfassen von medizinisch relevanten Messparametern (M, M₁, M₂),
und mit einer mit der Messanordnung (1) verbundenen oder verbindbaren Sende- und Empfangsanordnung (S),
wobei, dass die Sende- und Empfangsanordnung (S) zum Übermitteln der erfassten Messparameter (M, M₁, M₂) an eine aus einer Mehrzahl von verschiedenen Empfangs- und Sendestationen (E₁ bis E₆, E₁, Eₛ) ausgewählten Sendestation ausgebildet ist,
**dadurch gekennzeichnet, dass** die Sende- und Empfangsanordnung (S) zum wahlweise Übermitteln der erfassten Messparameter (M, M₁, M₂) über ein aus einer Mehrzahl von verschiedenen Übertragungsmedien ausgewählten Medium, wie insbesondere GSM oder UMTS oder über ein Satellitennetzwerk oder ein lokales Funknetzwerk ausgebildet ist.

12. Vorrichtung nach Anspruch 11, **dadurch gekennzeichnet, dass** die Sende- und Empfangsanordnung (S) zum Übertragen der ermittelten Messparameter (M, M₁, M₂) mit Radio Link Protocol (RLP) augebildet ist.

13. Vorrichtung nach einem der Ansprüche 11 oder 12, **dadurch gekennzeichnet, dass** die Messanordnung (1) eine Rechneranordnung (4) zum Codieren, Komprimieren oder zum Erzeugen eines Interleavings aufweisen.

14. Vorrichtung nach einem der Ansprüche 11 bis 13, **dadurch gekennzeichnet, dass** die Messanordnung (1) mit einem Speichermittel (2) zum zeitweisen Speichern der ermittelten Messparameter (m, M₁, M₂) versehen ist.

## Claims

1. Process for the remote monitoring of bodily functions, in particular heart and circulation functions, including the following stages:
Acquisition of medically relevant parameters (M, M₁, M₂) by means of sensors in a set-up of measuring instruments (1) on a body (K)
And transmission of the measurement parameters (M) recorded through a transmission and reception system (S) which is, or can be, connected to the set-up of measuring instruments (1), to an acquisition centre (10)
With the measurement parameters (M, M₁, M₂) being transmitted by radio to a reception and transmission station (E₁ to E₆, E₁, Eₛ) and from the latter to the acquisition centre (10), and
With the reception and transmission station (E₁) being selected from a number of reception and transmission stations (E₁ to E₆, E₁, Eₛ), distinguished by the fact that the wireless transmission is routed through a medium selected from a number of transmission media, in particular by means of a GSM or UMTS network, or by means of a satellite link or a local radio network.

2. Process as Claim 1, distinguished by the fact that the reception and transmission station (E) generating the strongest signal in the transmission and reception system (S) is automatically selected for the transmission of the measurement parameters (M, M₁, M₂.

3. Process as Claim 1, distinguished by the fact that the transmission is carried out using RLP.

4. Process as one of Claims 1 to 3, distinguished by the fact that the data to be transmitted are prepared before transmission by means of coding, compression and/or interleaving.

5. Process as one of Claims 1 to 4, distinguished by the fact that the measurement parameters are acquired by a set-up of measuring instruments (1) carried on the body (K), stored in the memory of the set-up of measuring instruments (1) in memory resources (3) and transmitted intermittently.

6. Process as one of Claims 1 to 4, distinguished by the fact that the measurement parameters are acquired by a set-up of measuring instruments (1) carried on the body (K) and are stored in the memory of the set-up of measuring instruments (1) in memory resources (3), and that the data saved in the cache memory (3) are interrogated by the acquisition centre (10).

7. Process as one of Claims 1 to 6, distinguished by the fact that at least one of the measurement parameters (M, M₁, M₂) from the group acquires and transmits electrical potentials in the body, substance concentrations in body fluids, pressures in the circulatory system, body temperatures, breathing parameters, skin resistance or body movements.

8. Process as one of Claims 1 to 7, distinguished by the fact that an alarm is triggered in the acquisition centre (10) and/or the set-up of measuring instruments (1) as soon as at least one of the parameters acquired (M, M₁, M₂) exceeds a pre-selected limit.

9. Process as one of Claims 1 to 8, distinguished by the fact that the measurement parameters transmitted (M, M₁, M₂) are saved in the acquisition centre (10) in a central memory resource (11).

10. Process as one of Claims 1 to 9, distinguished by the fact that when the strength of the signal of a first reception and transmission station (E₁) is reduced, and the signal of a second reception and transmission station (E₂) is enhanced during the data transmission, the link is transferred from the first reception and transmission station (E₁) to the second reception and transmission station (E₂).

11. Device for the remote monitoring of bodily functions, in particular heart and circulatory functions:
With a set-up of measuring instruments (1) with at least one sensor (2, 2₁, 2₂) for acquiring medically relevant measurement parameters (M, M₁, M₂)
And with a transmission and reception system (S) which is, or can be, linked to the set-up of measuring instruments (1)
With the transmission and reception system (S) for the transmission of the measurement parameters (M, M₁, M₂) acquired being developed for one of a number of different reception and transmission stations (E₁ to E₆, E₁, Eₛ)
Distinguished by the fact that the transmission and reception system (S) is developed for the selective transmission of the measurement parameters (M, M₁, M₂) acquired using a medium selected from a number of different transmission media, such as, in particular, GSM or UMTS, or using a satellite network or a local radio network

12. Device as Claim 11, distinguished by the fact that the transmission and reception system (S) for the transmission of the measurement parameters (M, M₁, M₂) is developed using radio link protocol (RLP).

13. Device as one of Claims 11 or 12, distinguished by the fact that the measurement system (1) has a computer unit (4) for coding, compressing or generating interleaving.

14. Device as one of Claims 11 to 13, distinguished by the fact that the set-up of measuring instruments (1) is provided with a memory resource (2) for the intermittent storage of the measurement parameters (M, M₁, M₂) acquired.

## Revendications

1. Procédé de télésurveillance de fonctions du corps, en particulier de fonctions du coeur et de la circulation, qui consiste
à enregistrer des paramètres médicaux importants (M, M₁, M₂) à l'aide de capteurs d'un dispositif de mesure (1), sur un corps (K),
et à transmettre les paramètres de mesure obtenus (M) à un point d'enregistrement (10) grâce à un émetteur-récepteur (S) relié ou apte à être relié au dispositif de mesure (1),
les paramètres de mesure (M, M₁, M₂) étant retransmis sans fil à une station de réception et d'émission (E₁ à E₆, E₁, Eₛ) et, à partir de celle-ci, au point d'enregistrement (10), et
la station de réception et d'émission (E₁) étant sélectionnée à partir d'une multiplicité de stations d'émission et de réception (E₁ à E₆, E₁, Eₛ) disponibles, **caractérisé en ce que** la transmission sans fil se fait, au choix, à l'aide d'un moyen sélectionné à partir d'une multiplicité de moyens de transmission, en particulier à l'aide d'un réseau GSM ou UMTS, ou à l'aide d'une liaison par satellite ou d'un réseau radio local.

2. Procédé selon la revendication 1, **caractérisé en ce que** pour transmettre les paramètres de mesure (M, M₁, M₂), c'est la station de réception et d'émission (E) générant le signal le plus fort dans l'émetteur-récepteur (S) qui est sélectionnée automatiquement.

3. Procédé selon la revendication 1, **caractérisé en ce que** la transmission se fait par RLP.

4. Procédé selon l'une des revendications 1 à 3, **caractérisé en ce que**, avant la transmission, les données à transmettre sont préparées par codage, compression et/ou entrelacement.

5. Procédé selon l'une des revendications 1 à 4, **caractérisé en ce que** les paramètres de mesure sont enregistrés par un dispositif de mesure (1) porté sur le corps (K), font l'objet d'un stockage intermédiaire dans le dispositif de mesure (1), dans des moyens de stockage (3), et sont retransmis par intermittence.

6. Procédé selon l'une des revendications 1 à 4, **caractérisé en ce que** les paramètres de mesure sont enregistrés par un dispositif de mesure (1) porté sur le corps (K) et font l'objet d'un stockage intermédiaire dans le dispositif de mesure (1), dans des moyens de stockage (3), et **en ce que** les données stockées dans la mémoire intermédiaire (3) sont consultées par le point d'enregistrement (10).

7. Procédé selon l'une des revendications 1 à 6, **caractérisé en ce que** l'un au moins des paramètres de mesure (M, M₁, M₂) est enregistré à partir du groupe constitué par les potentiels électriques dans le corps, les concentrations de matière dans les liquides du corps, les pressions dans le système de circulation, la température du corps, les paramètres de respiration, la résistance de la peau ou le mouvement du corps, et est retransmis.

8. Procédé selon l'une des revendications 1 à 7, **caractérisé en ce que** dans le point d'enregistrement (10) et/ou dans le dispositif de mesure (1), une alarme est déclenchée dès que l'un au moins des paramètres (M, M₁, M₂) enregistrés dépasse une valeur limite apte à être prédéfinie.

9. Procédé selon l'une des revendications 1 à 8, **caractérisé en ce que** les paramètres de mesure (M, M₁, M₂) retransmis sont stockés dans le point d'enregistrement (10), dans un moyen de stockage central (11).

10. Procédé selon l'une des revendications 1 à 9, **caractérisé en ce que** lors d'une diminution de l'intensité du signal d'une première station de réception et d'émission (E₁) et lors d'une augmentation du signal d'une seconde station de réception et d'émission (E₂) pendant la transmission des données, la liaison est transférée de la première (E₁) à la seconde (E₂).

11. Dispositif de télésurveillance de fonctions du corps, en particulier de fonctions du coeur et de la circulation,
comportant un dispositif de mesure (1) avec au moins un capteur (2, 2₁, 2₂) pour enregistrer des paramètres de mesure médicaux importants (M, M₁, M₂),
et un émetteur-récepteur (S) relié ou apte à être relié au dispositif de mesure (1),
l'émetteur-récepteur (S) étant conçu pour retransmettre les paramètres de mesure (M, M₁, M₂) enregistrés à une station d'émission sélectionnée à partir d'une multiplicité de stations de réception et d'émission différentes (E₁ à E₆, E₁, Eₛ),
**caractérisé en ce que** l'émetteur-récepteur (S) est conçu pour retransmettre au choix les paramètres de mesure (M, M₁, M₂) enregistrés, par l'intermédiaire d'un moyen sélectionné à partir d'une multiplicité de moyens de transmission différents, en particulier GSM ou UMTS, ou par l'intermédiaire d'un réseau satellite ou d'un réseau radio local.

12. Dispositif selon la revendication 11, **caractérisé en ce que** l'émetteur-récepteur (S) est conçu pour transmettre les paramètres de mesure enregistrés (M, M₁, M₂) avec le protocole hertzien (RLP).

13. Dispositif selon la revendication 11 ou 12, **caractérisé en ce que** le dispositif de mesure (1) comporte un ordinateur (4) pour le codage, la compression ou la production d'un entrelacement.

14. Dispositif selon l'une des revendications 11 à 13, **caractérisé en ce que** le dispositif de mesure (1) est pourvu d'un moyen de stockage (2) pour stocker par intermittence les paramètres de mesure obtenus (M, M₁, M₂).
